# EUROPEAN PATENT APPLICATION

(11) **EP 4 809 955 A1**
(43) Date of publication of application: **23.09.2026**
(21) Application number: 24891700.7
(22) Date of filing: 05.11.2024
(51) Int. Cl.: A61B 18/20, A61B 5/103, A61B 5/00, A61N 5/06, A61N 5/067, A61B 18/00

(54) **SKIN CARE TREATMENT DEVICE**

(30) Priority: 14.11.2023 KR 20230157447; 29.10.2024 KR 20240149603
(71) Applicant: APR CO., LTD., Seoul 05551 (KR)
(72) Inventor: LEE, Gyoun Jung, Seoul 05551 (KR); PARK, Seung Woo, Seoul 05551 (KR)
(74) Representative: Pestalozzi, Deborah
(86) International application number: PCT/KR2024/017264
(87) International publication number: WO 2025/105753

(57) **Abstract**

Disclosed is a skin care treatment device. The skin care treatment device according to an embodiment of the present invention includes: a main body; a suction header having a suction port, which comes into contact with skin, at an end portion protruding outward from the main body; a light irradiation module for irradiating the user's skin, which comes into contact with the suction port, with light that is generated when power is applied; a suction module that is provided inside the main body and applies negative pressure for raising a treatment site of the skin, which comes into contact with the suction port, to the suction port when power is applied; at least one optical sensor for sensing and measuring light that enters through an optical path formed at the end portion of the suction port which comes into contact with the skin; and a control unit electrically connected to the light irradiation module and the suction module. The control unit can selectively control the operation timing of the suction module by determining the contact state between the suction port and the skin on the basis of a measurement value detected by the optical sensor.

## Description

### TECHNICAL FIELD

The present invention relates to a skin care treatment device, and more particularly, to a skin care treatment device capable of determining a contact state between a user's skin and a suction port on the basis of a skin color or a brightness of light related to light measured in an area to be suctioned when the skin comes into contact with the user's skin.

### BACKGROUND

In general, a depilator is a device for removing body hair grown on the skin of a human body, that is, hair, head hair, and the like.

Traditional depilators include razors that cut body hair by using razor blades, and grain-thresher-type forced depilators that forcibly pull out body hair to remove hair roots. However, although such hair removal methods are convenient, they have a disadvantage in that the duration of the effect is short.

Accordingly, in recent years, a hair removal method using a laser has drawn attention as a fundamental solution for preventing hair growth.

The principle of laser hair removal is to damage cells that generate hair so that hair does not grow again from a hair follicle. A hair follicle is a skin organ that produces hair, and when melanocytes disappear from the hair follicle, the hair follicle can no longer produce hair.

That is, by using optical energy in the form of pulses, the optical energy is absorbed into melanin chromophores of the skin according to the principle of selective photothermolysis and is converted into thermal energy, and such thermal energy is delivered to the hair follicles to remove unwanted hair.

(Patent Document 1) KR 10-1784536 B1

Patent Document 1 discloses a device for cosmetically treating skin by mounting a vacuum module and forcibly suctioning the skin with negative pressure by means of a suction force of a vacuum pump.

In Patent Document 1, in order for the skin in contact with an end portion of the suction port to be forcibly suctioned by the negative pressure, which is the vacuum suction force of the vacuum module mounted in the cosmetic treatment device, the skin may be efficiently forcibly suctioned by the negative pressure generated during operation of the vacuum module in a state in which an open end of the suction port is evenly brought into contact with the skin as a whole.

However, it is difficult to visually check whether the open end of the suction port is in a state of being uniformly brought into contact with the user's irregularly shaped skin as a whole. When the open end of the suction port is non-uniformly and partially brought into contact with the user's skin so that a gap is formed, skin suction by the vacuum suction force generated by the vacuum module is not smoothly performed, so that cosmetic treatment of the skin becomes impossible or is performed inefficiently.

In addition, in order to initially maintain a close contact state between the user's skin and the suction port before the vacuum module operates, the user has to press the end portion of the suction port against the skin to forcibly bring the suction port into close contact with the skin, thereby causing inconvenience in use.

Further, although a touch sensor for detecting whether contact with the skin is made may be provided at the open end of the suction port to confirm a contact state between the skin and the suction port, it is fundamentally impossible to detect, by the touch sensor alone, a state in which the entire open end of the suction port is brought into contact with the skin.

### DISCLOSURE

### TECHNICAL PROBLEM

The present invention has been made to solve the above-described problems, and an object of the present invention is to provide a skin care treatment device configured to determine a contact state between a user's skin and a suction port to which negative pressure is applied on the basis of a brightness value of a skin color and to control an operation timing of a suction module.

Another object of the present invention is to provide a skin care treatment device capable of controlling an output intensity of a suction module on the basis of a skin color measured in a skin region of a user.

The technical problems to be achieved in the present invention are not limited to the technical problems mentioned above, and other technical problems not mentioned will be clearly understood by those of ordinary skill in the art from the following description.

### TECHNICAL SOLUTION

According to an aspect of the present invention, a skin care treatment device is provided, including: a main body;
a suction header having, at an end portion protruding outward from the main body, a suction port configured to contact skin; a light irradiation module configured to irradiate, toward a user's skin contacting the suction port, light generated when power is applied; a suction module provided inside the main body and configured to apply, toward the suction port, negative pressure for raising a treatment site of the skin contacting the suction port when power is applied; at least one optical sensor configured to sense and measure light introduced inward through an optical path formed at an end portion of the suction port contacting the skin; and a control unit electrically connected to the light irradiation module and the suction module, respectively, wherein the control unit is configured to determine a contact state between the suction port and the skin based on a measured value sensed by the optical sensor and selectively control an operation timing of the suction module.

In this case, the optical sensor may be a color sensor configured to sense a skin color of a skin region related to light introduced through the optical path and convert color information into an electrical signal.

In this case, the optical sensor may be an illuminance sensor configured to sense brightness of light introduced through the optical path and convert the brightness into an electrical signal according to an amount of the sensed light.

In this case, the skin care treatment device may further include at least one auxiliary optical sensor provided on an inner wall of the suction port corresponding to a suction space formed inside the suction port, and the auxiliary optical sensor may be one of a color sensor configured to sense a skin color of a skin region related to light introduced into the suction space and convert color information into an electrical signal, and an illuminance sensor configured to sense brightness of light introduced into the suction space and convert the brightness into an electrical signal according to an amount of the sensed light.

In this case, the control unit may be configured to control an output intensity of the light irradiation module based on the skin color sensed and measured by the color sensor.

In this case, the suction port may include at least one touch sensor configured to generate a contact signal upon contact with the skin at an end portion of the suction port and transmit the generated contact signal to the control unit.

### ADVANTAGEOUS EFFECT

According to the above configuration, the skin care treatment device according to the present invention can accurately and easily confirm a contact state between skin and a suction port based on real-time measured values of an optical sensor that senses light provided through an optical path provided in the suction port contacting the skin and measures a brightness value of a skin color or a brightness value of light. Therefore, the skin care treatment device can precisely and accurately control an operation timing of a suction module that applies negative pressure to a suction space and can efficiently raise skin in a skin region by the negative pressure applied from the suction module without unnecessary power loss.

In addition, by accurately determining a skin color based on measured values of the optical sensor or an auxiliary optical sensor that senses the skin color in the skin region, the skin care treatment device can adjust and irradiate an output intensity of laser light for hair removal according to the determined skin color or a brightness of the skin color, or can stop irradiation of the laser light. Accordingly, hair removal with respect to a skin treatment site can be safely performed without concern of skin damage or skin burns during hair removal, thereby improving user satisfaction and enhancing hair removal efficiency.

Advantageous effects of the present invention are not limited to the above-described effects, and it should be understood that the present invention includes all effects that can be inferred from the configurations described in the detailed description or claims.

### BRIEF DESCRIPTION OF THE DRAWINGS

FIG. 1 is an overall perspective view illustrating a skin care treatment device according to an embodiment of the present invention.
FIG. 2 is a schematic view illustrating an installation state of a suction module applied to the skin care treatment device according to an embodiment of the present invention.
FIG. 3 is a detailed view illustrating a suction port of a suction header applied to the skin care treatment device according to an embodiment of the present invention.
FIG. 4 is a cross-sectional view illustrating a suction port of a suction header applied to the skin care treatment device according to an embodiment of the present invention.
FIG. 5 is a detailed view illustrating a suction port of a suction header applied to a skin care treatment device according to another embodiment of the present invention.
FIG. 6 is a cross-sectional view illustrating a suction port of a suction header applied to a skin care treatment device according to another embodiment of the present invention.
FIGS. 7A and 7B are schematic views illustrating, in a state in which the suction header of the skin care treatment device according to an embodiment of the present invention is in contact with skin, a poor-contact state in which a gap is formed and a good-contact state in which no gap is formed.
FIG. 8 is a Fitzpatrick skin classification table, which is a criterion for determining a skin color measured by a color sensor of the skin care treatment device according to an embodiment of the present invention.
FIGS. 9A, 9B, and 9C are operation state views illustrating close contact, suction, and exhaust in the skin care treatment device according to an embodiment of the present invention.
FIG. 10 is an operation flowchart of the skin care treatment device according to an embodiment of the present invention.

### MODES OF THE INVENTION

Hereinafter, exemplary embodiments of the present invention will be described in detail with reference to the accompanying drawings so that those of ordinary skill in the art can readily implement the present invention. The present invention may be embodied in many different forms and is not limited to the embodiments set forth herein. Portions irrelevant to the description have been omitted for clarity of explanation of the present invention, and throughout the specification, the same reference numerals are used to denote identical or similar components.

The words and terms used in the present specification and claims should not be interpreted as being limited to their ordinary or dictionary meanings, but should be construed as having meanings and concepts consistent with the technical spirit of the present invention, in accordance with the principle that an inventor may define terms and concepts to best describe their invention.

Accordingly, the embodiments described in the present specification and the configurations shown in the drawings correspond to preferred embodiments of the present invention, and do not represent all the technical spirit of the present invention, so the configurations may have various examples of equivalent and modification that can replace them at the time of filing the present invention.

It should be understood that the terms "comprise or include" or "have" or the like when used in this specification, are intended to describe the presence of stated features, numbers, steps, operations, elements, components and/or a combination thereof but not preclude the possibility of the presence or addition of one or more other features, numbers, steps, operations, elements, components, or a combination thereof.

The presence of an element in/on "front", "rear", "upper or above or top" or "lower or below or bottom" of another element includes not only being disposed in/on "front", "rear", "upper or above or top" or "lower or below or bottom" directly in contact with other elements, but also cases in which another element being disposed in the middle, unless otherwise specified. In addition, unless otherwise specified, that an element is "connected" to another element includes not only direct connection to each other but also indirect connection to each other.

Hereinafter, a skin care treatment device according to a preferred embodiment of the present invention will be described with reference to the drawings.

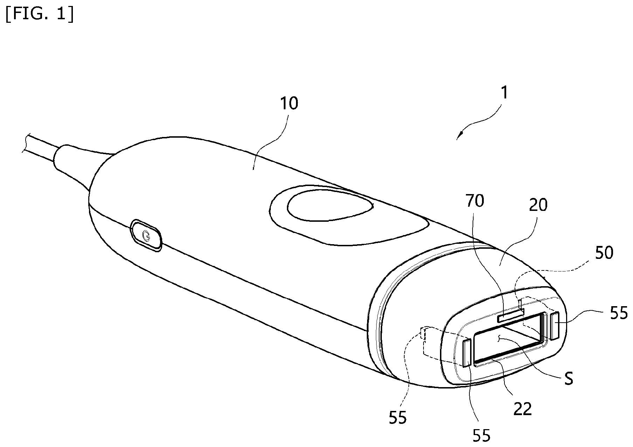

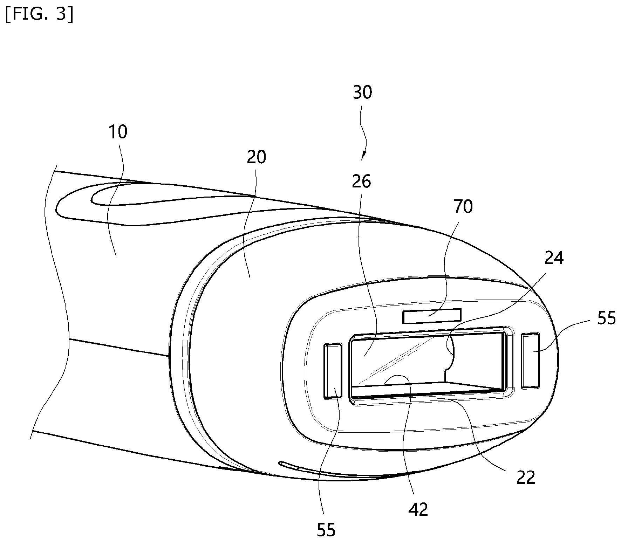

As illustrated in FIGS. 1, 2, 3, and 4, the skin care treatment device 1 according to a preferred embodiment of the present invention may include a main body 10, a suction header 20, a light irradiation module 30, a suction module 40, a control unit, and an optical sensor 50.

Hereinafter, in describing with reference to the drawings, a direction in which a suction port 22 of the suction header 20 of the skin care treatment device 1 is directed is defined as a forward direction, and an opposite direction is defined as a rearward direction, and a space having a predetermined size formed inside the suction port 22 when the suction port 22 comes into contact with skin is defined as a suction space S.

Such a skin care treatment device 1 may forcibly raise the skin to a predetermined height in the suction space S inside the suction port 22 by applying predetermined negative pressure to the suction port 22 of the suction header 20 that directly comes into contact with the user's skin.

Subsequently, by bringing the raised skin closer to the light irradiation module 30, the skin care treatment device 1 may improve light irradiation efficiency for hair removal and perform predetermined skin care treatment effects such as massage.

Such application of negative pressure may be performed by at least one suction module 40.

In this case, the skin care treatment device 1 according to an embodiment of the present invention may include at least one optical sensor 50 installed adjacent to the suction space.

The optical sensor 50 is a sensor member that senses light introduced inward through an optical path 55 formed at an end portion of the suction port that comes into contact with the skin, measures brightness of a skin color corresponding to a skin tone or brightness of the light corresponding to illuminance, and optically processes the same.

The optical sensor 50 may be a color sensor that senses a skin color of a skin region related to the light introduced inward through the optical path 55 and converts color information into an electrical signal, or an illuminance sensor that senses brightness of the light introduced inward through the optical path 55 and converts the sensed light into an electrical signal according to an amount of the sensed light.

In addition, the control unit may selectively control an operation timing of the suction module 40 by determining a contact state between an end portion of the suction port 22 and corresponding skin on the basis of a measured brightness value of the skin color sensed by the optical sensor 50 or a measured brightness value of the light sensed by the optical sensor 50.

In a complete contact state in which the end portion of the suction port 22 is entirely in close contact with the skin, the suction module 40 is operated to generate negative pressure in the suction space, so that the generated negative pressure may be applied to a skin region to be skin-treated, thereby raising the skin to a predetermined height.

Accordingly, according to the skin care treatment device 1 according to an embodiment of the present invention, it is possible to determine whether complete contact between the skin and the suction port, which is an essential prerequisite before applying negative pressure to and suctioning the skin, is achieved, thereby maximizing a skin care treatment effect intended to be achieved by the skin care treatment device 1.

Hereinafter, the respective components of the skin care treatment device according to an embodiment of the present invention will be described in detail.

The skin care treatment device 1 according to an embodiment of the present invention includes a main body 10.

As illustrated in FIGS. 1, 2, 3, and 4, the main body 10 may serve as a casing for accommodating other components and may also be provided such that a user can grip the main body 10 to operate and control the skin care treatment device 1.

The main body 10 may have an internal space of a predetermined size for accommodating the suction module 40 and may be provided in a substantially straight bar shape, or may have a form including a handle that can be gripped by a user's hand.

A display for indicating a control state of the skin care treatment device 1 and a button member or a touch screen through which a signal for controlling the skin care treatment device 1 can be input may be provided on an outer surface of the main body 10.

Meanwhile, a shape of the main body 10 is not particularly limited, and may be appropriately modified according to characteristics of other components accommodated therein or a use environment or the like.

The main body 10 may include the suction header 20 having the suction port 22, an end portion of which comes into contact with the skin, may include at least one suction module 40 disposed in an internal space of the main body 10 and generating negative pressure, which is a suction force, when power is applied, and may include at least one light irradiation module 30 disposed at an end portion of the main body 10 adjacent to the suction module 40 and generating laser light when power is applied.

Here, the suction header 20 may be detachably assembled to the end portion of the main body.

As illustrated in FIGS. 1, 2, 3, and 4, the suction header 20 may extend from the main body 10 and be integrally provided therewith or detachably assembled thereto, and may include a suction port 22 for applying negative pressure to a skin region formed while directly contacting the skin.

Here, the suction header 20 may be provided in a funnel shape having a diameter decreasing toward the front.

The suction header 20 includes the suction port 22 opened forward, and at least one communication hole 24 may be formed through an inner side of the suction port 22 to be in fluid communication with a vacuum suction line 42 extending from the suction module 40, such that negative pressure, which is a suction force, is transmitted to the suction space S.

An inner region of the suction header 20 corresponding to the suction port 22 may include a transparent window 26 corresponding to a light source 32 of the light irradiation module 30 and configured to transmit treatment light such as laser light toward the skin without loss.

Accordingly, a suction space to which negative pressure is applied so as to raise, to a predetermined height, the skin contacting the suction port may be formed between the suction port contacting the skin and the transparent wall corresponding to the light source.

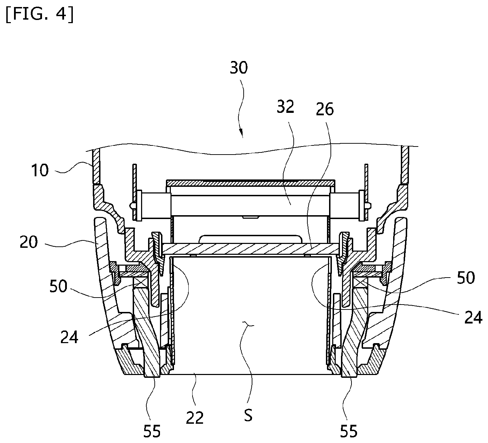

The light irradiation module 30 may include, as illustrated in FIG. 4, at least one light source 32 configured to directly and intensively irradiate the raised skin, which is raised by the suction force of the suction module 40, with laser light, which is treatment light generated when power is applied.

The light source 32 provided in the light irradiation module 30 may be an IPL (Intense Pulsed Light) laser light source or may include a flash lamp, the IPL laser light source irradiating the raised skin with laser light having a wavelength band of 560 nm to 1100 nm when power is applied and performing a hair removal function of removing hair by necrosing hair follicles and surrounding portions of the hair follicles by means of the laser light having high-output energy.

The light source 32 may include a plurality of LEDs that generate light having high-output energy in a wavelength band of 560 nm to 1100 nm when power is applied, and a substrate on which the plurality of LEDs are mounted may be installed on an inner surface of a sealed end portion of the main body 10.

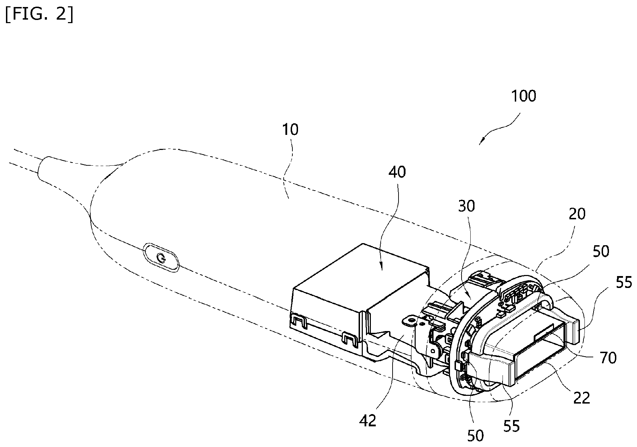

As illustrated in FIGS. 2, 3, and 4, the suction module 40 may be disposed inside the main body 10 and may include at least one suction source configured to generate suction force when power is applied so as to apply negative pressure to the skin through the suction space S.

The suction module 40 is configured to generate negative pressure in the suction space S formed inside the suction port 22 of the suction header 20, and may be, for example, a pumping motor or the like capable of generating negative pressure by forcibly suctioning outside air when power is applied.

A vacuum suction line 42 extending from the suction module 40 is in fluid communication with a communication hole 24 formed through one side or both sides of the suction port 22, and an exhaust line (not shown) extending from the suction module 40 may be in fluid communication with an exhaust hole formed through an outer surface of the main body.

Control of the negative pressure of the suction module 40 may be configured to be controlled by the control unit.

In this case, the suction module 40 may be configured such that an intensity of the negative pressure can be adjusted by operation of a button provided outside the main body, and the suction module 40 may be configured such that an output corresponding to the suction force is variable.

As illustrated in FIGS. 1, 2, 3, and 4, the optical sensor 50 is at least one sensor member provided inside the suction header 20 and configured to sense and measure light introduced inward through the optical path 55 provided at an end portion of the suction port 22 that comes into contact with the skin.

The optical sensor 50 may be provided as a color sensor that senses a skin color of a skin region corresponding to the optical path 55 and converts color brightness and a skin tone, which are color information, into an electrical signal as a measured value, or may be provided as an illuminance sensor that measures an amount of light obtained from a region corresponding to the optical path 55 and converts the same into an electrical signal as a measured value according to brightness of the light.

The optical path 55 may be provided as an optical guide made of a light-transmitting resin material, with an optical input end, which is a front end, disposed in an exposure hole formed through an end portion of the suction port 22 that comes into contact with the skin and with the optical sensor 50 disposed at an optical output end, which is a rear end, or may be provided as an empty space extending from the exposure hole formed through the end portion of the suction port 22 that comes into contact with the skin to the optical sensor.

In this case, the optical sensors 50 may be arranged in a plurality of pairs facing each other at the end portion of the suction port 22, or may be arranged in plural at equal intervals in a circumferential direction about a center of the suction port 22.

An end portion of the suction port 22 may include, as illustrated in FIGS. 1, 2, 3, 4, 5, and 6, at least one touch sensor 70 configured to generate a signal upon contact with the skin and transmit the generated contact signal to the control unit.

Such a touch sensor 70 may generate a contact signal while coming into prior contact with the skin in a process of bringing the end portion of the suction port 22 close to the user's skin, and the contact signal may be transmitted to the control unit.

Based on the contact signal of the touch sensor 70, the control unit may control the optical sensor 50 and the auxiliary optical sensor 60, which are provided as the color sensor or the illuminance sensor together with the light irradiation module 30 and the suction module 40, to be switched from an idle mode in which power is completely cut off to a standby mode in which standby power is applied and an operation is awaited.

The control unit may be electrically connected to the light irradiation module 30 and the suction module 40, respectively, to transmit control signals, and may be accommodated in an internal space of the main body 10 or an internal space of the suction header 20 so as to be electrically connected to the color sensor or the illuminance sensor constituting the optical sensor 50 and receive information corresponding to a brightness value of a skin color or a brightness value of light.

Such a control unit may be implemented by a processor, a central processing unit (CPU), a controller, a logic arithmetic unit, an arithmetic logic circuit, a digital signal processor, a microcomputer, an FPGA, a system on chip (SoC), a programmable logic unit, a microprocessor, or the like.

In this case, in the present specification, being electrically connected may include not only being connected by an electrical line so as to be capable of electrical conduction, but also being connected by a known wireless communication method.

Accordingly, the control unit according to the present embodiment may be configured to acquire information corresponding to a predetermined measured value from the optical sensor 50 or the auxiliary optical sensor 60, each formed as the color sensor or the illuminance sensor, and to control operation of the light irradiation module 30 and operation of the suction module 40 on the basis of the acquired information.

Meanwhile, when the optical sensor 50 is provided as a color sensor, the color sensor is a sensor that senses, as a color, light obtained through the optical path 55, measures in real time a skin color corresponding to a skin tone and a brightness value of the sensed skin color, and transmits information corresponding to the measured values to the control unit.

Such a color sensor detects R (red), G (green), and B (blue), which are the three primary colors of light, and may detect the skin color corresponding to the skin tone and obtain a brightness value of the skin color by receiving light through a photodiode and detecting RGB values.

For example, when an amount of light introduced through the optical path 55 is large and thus bright, a resistance value related to an amount of color brightness measured by the color sensor decreases, and when the amount of light introduced through the optical path 55 is small and thus relatively dark, the resistance value related to the amount of color brightness measured by the color sensor increases, such that a brightness value of the color may be obtained based on the varying resistance value.

And, a light source that provides brightness of light to be sensed and measured by the color sensor may be natural light such as sunlight when a location where the user uses the skin care treatment device is outdoors, and may be artificial light such as indoor illumination when the location where the user uses the skin care treatment device is indoors.

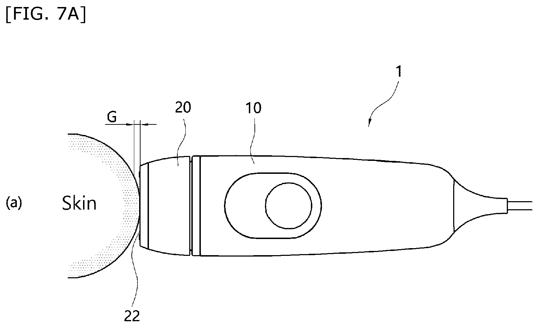

As illustrated in FIG. 7A, in a process in which the end portion of the suction port 22 comes into contact with skin having a substantially curved surface, an input end of the optical path 55 may be completely exposed to the outside at the end portion of the suction port 22, or may partially contact the skin and thus be partially exposed to the outside, while forming a gap G between the end portion of the suction port 22 and the skin.

In this case, natural light or artificial light may enter and be sensed by the color sensor, which is the optical sensor, through the input end of the optical path 55 that is completely exposed or partially exposed.

A brightness value of the skin color sensed and measured by the color sensor may be set to "10," which is a brightest exposed state, according to an amount of light obtained through the optical path, or may be set to a measured value between "1" and "9," which is lower than the maximum value, and, based thereon, the control unit may determine that an entire edge, which is the end portion of the suction port 22, is in a non-contact state in which the entire edge is not completely in contact with the skin or in an incomplete close-contact state in which the entire edge is not completely closely adhered.

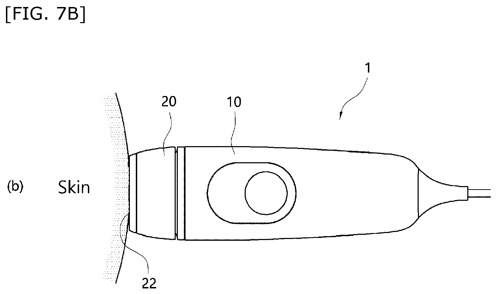

In contrast, as illustrated in FIG. 7B, the end portion of the suction port 22 may come into contact with the skin without forming the gap G, and the input end of the optical path 55 may be completely covered by and closely adhere to the skin.

In this case, natural light or artificial light is not introduced at all into the optical path 55 covered by the skin and is thus not sensed by the color sensor, which is the optical sensor.

A brightness value of the skin color measured by the color sensor may be set to "0," and, based thereon, the control unit may determine that the optical path 55 is in a dark state in which no light is introduced at all, and may determine that the entire edge, which is the end portion of the suction port 22, is in a state of complete contact with the skin.

Accordingly, when the brightness value of the skin color measured by the color sensor provided as the optical sensor is measured as a brightness value other than "0," the control unit may determine that the entire edge, which is the end portion of the suction port 22, is in a state of incomplete close contact with the skin or in a non-contact state, and may control the light irradiation module 30 and the suction module 40 to be maintained in a standby mode state.

In contrast, when the brightness value of the skin color measured by the color sensor is measured as "0," the control unit may determine that the end portion of the suction port 22 is in close contact with the skin, and may control the suction module 40 to start operation so as to apply negative pressure to the skin and raise the skin.

Meanwhile, information corresponding to the measured value of the skin color measured by the color sensor may be numerically classified and set as the skin color corresponding to the skin tone on the basis of a Fitzpatrick skin classification table for determining brightness of the skin according to hue and lightness of the skin.

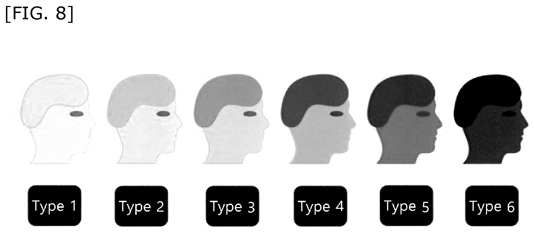

That is, as illustrated in FIG. 8, the Fitzpatrick skin classification table classifies skin colors into six types according to skin color and a skin response to sunlight, and classifies a very light skin color as Type 1, a light skin color as Type 2, a light-to-medium skin color as Type 3, a medium skin color as Type 4, a dark skin color as Type 5, and a very dark skin color as Type 6.

Accordingly, the control unit may compare and determine the user's skin color sensed and measured by the optical sensor 50 provided as the color sensor with the six skin colors classified in the Fitzpatrick skin classification table, thereby selecting and determining the user's skin color as one of Types 1 to 6.

In this case, when the control unit determines that the user's skin color measured by the color sensor is Type 5 corresponding to a dark skin color or Type 6 corresponding to a very dark skin color, the control unit may reduce an intensity of laser light for hair removal output from the light irradiation module 30 and irradiated toward the skin, or may stop light irradiation.

This is because hair removal is performed based on absorption of optical energy by melanin chromophores of the skin, and thus, the darker the skin color, such as Type 5 or Type 6, the higher the optical energy absorption rate, so that the skin may be damaged by the laser light.

In addition, when the optical sensor 50 is provided as an illuminance sensor, the illuminance sensor is a sensor member that senses an amount of light obtained through the optical path 55, measures, in real time, brightness of the light as internal illuminance, and transmits information corresponding to the measured value to the control unit.

Such an illuminance sensor is a sensor for measuring brightness of light, which is the amount of sensed light, and a resistance value thereof varies according to the amount of light obtained through the optical path, through which a brightness value of the light may be obtained.

For example, when an amount of light introduced into the optical path 55 is large, a resistance value related to an amount of brightness of light measured by the illuminance sensor decreases, and when the amount of light introduced into the optical path 55 is small, the resistance value related to the amount of brightness of light measured by the illuminance sensor increases, such that the brightness value of the light may be obtained based on the varying resistance value.

And, a light source that provides brightness of light to be measured by the illuminance sensor may be natural light such as sunlight when a location where the user uses the skin care treatment device is outdoors, and may be artificial light such as indoor lighting when the location where the user uses the skin care treatment device is indoors.

As illustrated in FIG. 7A, in a process in which the end portion of the suction port 22 comes into contact with skin having a substantially curved surface, an input end of the optical path 55 may be completely exposed to the outside at the end portion of the suction port 22, or may partially contact the skin and thus be partially exposed to the outside, while forming a gap G between the end portion of the suction port 22 and the skin.

In this case, natural light or artificial light may enter and be sensed by the illuminance sensor, which is the optical sensor, through the input end of the optical path 55 that is completely exposed or partially exposed.

A brightness value of the light sensed and measured by the illuminance sensor may be set to "10," which is a brightest exposed state, according to an amount of light obtained through the optical path 55, or may be set to a measured value between "1" and "9," which is lower than the maximum value, and, based thereon, the control unit may determine that an entire edge, which is the end portion of the suction port 22, is in a non-contact state in which the entire edge is not completely in contact with the skin or in an incomplete close-contact state in which the entire edge is not completely closely adhered.

In contrast, as illustrated in FIG. 7B, the end portion of the suction port 22 may come into contact with the skin without forming the gap G, and the input end of the optical path 55 may be completely covered by and closely adhere to the skin.

In this case, natural light or artificial light is not introduced at all into the optical path 55 covered by the skin and is thus not sensed by the illuminance sensor, which is the optical sensor.

A brightness value of the light measured by the illuminance sensor may be set to "0," and, based thereon, the control unit may determine that the optical path 55 is in a dark state in which no light is introduced at all, and may determine that the entire edge, which is the end portion of the suction port 22, is in a state of complete contact with the skin.

Accordingly, when the brightness value of the light measured by the illuminance sensor provided as the optical sensor is measured as a brightness value other than "0," the control unit may determine that the entire edge, which is the end portion of the suction port 22, is in a state of incomplete close contact with the skin or in a non-contact state, and may control the light irradiation module 30 and the suction module 40 to be maintained in a standby mode state.

In contrast, when the brightness value of the light measured by the illuminance sensor is measured as "0," the control unit may determine that the end portion of the suction port 22 is in close contact with the skin and may control the suction module 40 to start operation so as to apply negative pressure to the skin and raise the skin.

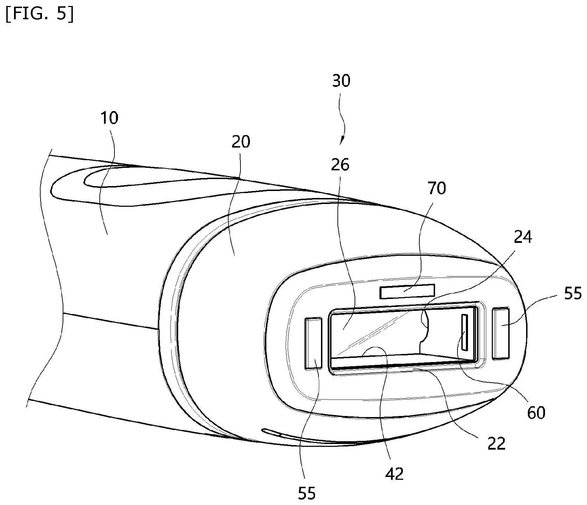

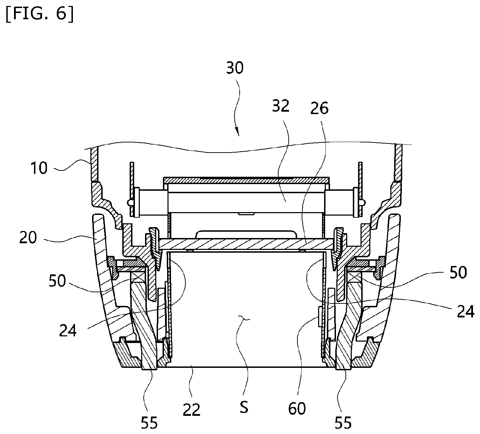

Meanwhile, as illustrated in FIGS. 5 and 6, the skin care treatment device may further include at least one auxiliary optical sensor 60 provided on an inner wall of the suction port 22 corresponding to the suction space S formed inside the suction port 22.

Here, the auxiliary optical sensor 60 may be selectively provided as either a color sensor that determines a skin color of the skin and measures brightness of the skin color based on light introduced into the suction space S, or an illuminance sensor that measures brightness of light introduced into the suction space S.

The optical sensor 50 and the auxiliary optical sensor 60 may be provided as different types of sensors or as the same type of sensor.

In addition, the auxiliary optical sensor 60 may be provided as an independent sensor member that senses and measures light introduced into the suction space S without installation of the optical sensor 50 exposing the input end of the optical path at an end portion of the suction port.

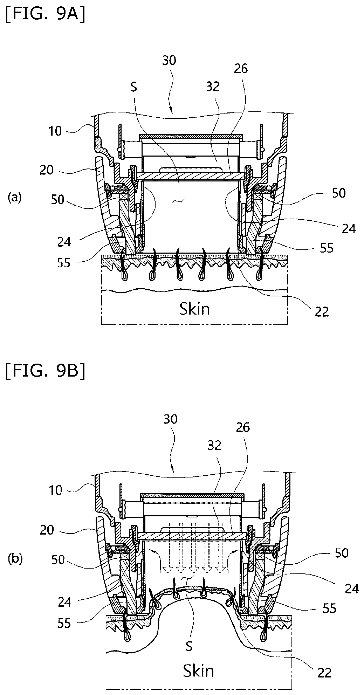

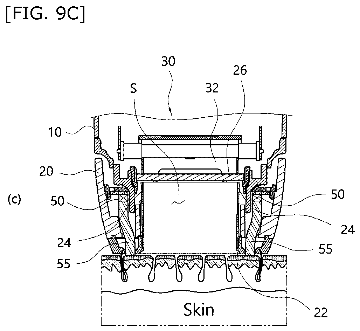

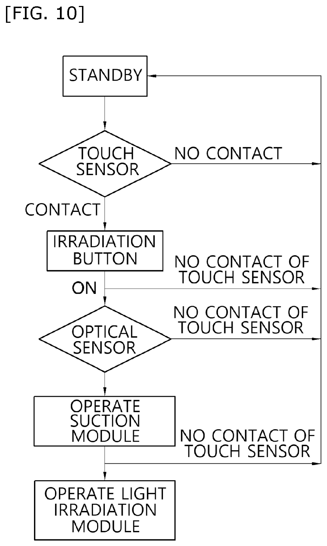

FIGS. 9A, 9B, and 9C are operation state views illustrating close contact, suction, and exhaust in the skin care treatment device according to an embodiment of the present invention, and FIG. 10 is an operation flowchart of the skin care treatment device according to an embodiment of the present invention.

First, in order to perform a cosmetic procedure of raising, by negative pressure, skin to be depilated by using the skin care treatment device according to an embodiment of the present invention and then removing hair from the raised skin with laser light, the suction header 20 is first positioned to correspond to and face the skin to be depilated.

In this state, when the suction port 22 is moved close so that an end portion of the suction port 22 comes into contact with the skin, the touch sensor 70 provided at an end portion of the suction header first comes into contact with the skin and generates a contact signal, and the contact signal of the touch sensor is transmitted to the control unit.

In this case, standby power is applied to the light irradiation module 30 and the suction module 40 in response to the contact signal of the touch sensor, so that the light irradiation module 30 and the suction module 40 are switched to a standby mode, and standby power may also be applied to the optical sensor 50 and the auxiliary optical sensor 60, each provided as one of the color sensor and the illuminance sensor, so that the optical sensor 50 and the auxiliary optical sensor 60 are switched to a standby mode for measuring the skin color and brightness of the skin color or brightness of light.

Such a standby mode may be performed by a pressing operation of a start button provided on the main body.

And, in a state in which the end portion of the suction port 22 is in contact with the skin, the optical sensor 50 and the auxiliary optical sensor 60, each corresponding to the color sensor or the illuminance sensor, may measure a brightness value of the skin color related to light introduced and sensed through the optical path 55 or light introduced into the suction space S, or may measure a brightness value of the light.

That is, as illustrated in FIG. 7A, when light is introduced through the input end of the optical path exposed at the end portion of the suction port 22 or light is introduced into the suction space, a brightness value of the skin color or a brightness value of the light measured by the optical sensor 50 and the auxiliary optical sensor 60, each provided as one of the color sensor and the illuminance sensor, is measured as an arbitrary measured value corresponding to "10," which is a non-contact state, or between "1" and "9," which is an incomplete contact state, excluding "0," which is a dark state, and is transmitted to the control unit.

In contrast, when the input end of the optical path exposed at the end portion of the suction port 22 is completely covered by the skin or when the suction space is completely closed, as illustrated in FIGS. 7B and 9A, no gap G is formed between the end portion of the suction port 22 and the skin, and no external light is introduced at all through the input end of the optical path 55 or the suction space, and therefore a brightness value of the skin color or a brightness value of the light measured by the optical sensor 50 or the auxiliary optical sensor 60, each provided as one of the color sensor and the illuminance sensor, is measured as a measured value of "0," which is a dark state, and is transmitted to the control unit.

In this case, the control unit may determine, based on information corresponding to the measured values transmitted from the optical sensor 50 and the auxiliary optical sensor 60, that the end portion of the suction port 22 is in an incomplete contact state in which the end portion partially contacts the skin, or may determine that the end portion of the suction port 22 is in a complete close-contact state in which the end portion is entirely and evenly in contact with the skin.

When the control unit determines the incomplete contact state, the suction module 40 maintains a standby state, whereas, when the control unit determines the complete close-contact state, the control unit applies power to the suction module 40 so as to generate the suction force and cause the suction module 40 to apply negative pressure to the suction space S, thereby raising the skin in contact with the end portion of the suction port.

Accordingly, based on the contact signal of the touch sensor and the brightness value of the skin color or the brightness value of the light in the suction space measured by the optical sensor 50 and the auxiliary optical sensor 60, it is possible to accurately confirm a close-contact state in which the end portion of the suction port is completely in contact with the skin, which is a treatment site to be depilated, and at the same time perform operation of the suction module 40 without suction-force loss.

As illustrated in FIG. 9B, when the suction module 40 is operated in a state in which the end portion of the suction port 22 is in complete close contact with the skin and negative pressure is applied to the suction space S, the skin corresponding to the suction space S may be convexly raised to a predetermined height toward the light irradiation module 30.

Subsequently, hair removal may be performed by the laser light generated during operation of the light irradiation module 30 being irradiated toward the skin raised to the predetermined height by the negative pressure applied to the suction space S.

In this case, after determining the skin color as one of six types based on the measured value of the brightness value of the skin color measured by the optical sensor 50 or the auxiliary optical sensor 60, each including the color sensor, an intensity of the laser light irradiated from the light irradiation module 30 toward the skin may be controlled.

That is, when the skin color measured by the color sensor is determined by the control unit as Type 1 to Type 4 among Types 1 to 6, hair removal by the laser light may be performed without concern of skin damage or skin burns by irradiating the raised skin with laser light having an output controlled to a preset light intensity corresponding to a very light skin color, a light skin color, a light-to-medium skin color, or a medium skin color, or by irradiating the raised skin with laser light having an output controlled to a preset uniform light intensity.

And, when an upper portion of the skin raised by the negative pressure of the suction module 40 becomes wide and flat, it is preferable to maintain the negative pressure applied by the suction module 40 for a predetermined period of time during which hair removal by the laser light of the light irradiation module 30 is performed.

In contrast, when the brightness value of the skin color measured by the color sensor is determined by the control unit as Type 5 or Type 6 among Types 1 to 6, power supply to the light irradiation module may be controlled not to be performed because skin damage and skin burns may occur when laser light is irradiated onto skin having a dark skin color or a very dark skin color.

In this case, the color sensor for measuring a skin color of a skin region to be depilated may measure the skin color of the skin region before an end portion of the suction port 22 comes into contact with the skin, based on external light such as natural light or artificial light, or may measure the skin color of the skin region in a dark state in which the end portion of the suction port is in complete close contact with the skin, based on light from a lighting light source that is not shown.

Finally, as illustrated in FIG. 9C, when hair removal with respect to the treatment site is completed by irradiating the treatment site of the skin raised by the negative pressure of the suction module 40 with the laser light of the light irradiation module 30, the negative pressure in the suction space S may be eliminated by selectively opening an exhaust port provided in the main body 10 so as to be in communication with an exhaust line of the suction module 40, and thus the skin raised by the negative pressure is returned to its original state and, at the same time, all hair removal processes are terminated.

Although exemplary embodiments of the present invention have been described, the spirit of the present invention is not limited to the embodiments set forth herein. Those of ordinary skill in the art who understand the spirit of the present invention may easily propose other embodiments through supplement, change, removal, addition, etc. of elements within the same scope of spirit, but the embodiments will be also within the scope of the present invention.

**[Description of Symbols]**

| | | | |
|---|---|---|---|
| 10: | main body | 20: | suction header |
| 22: | suction port | 24: | communication hole |
| 30: | light irradiation module | 32: | light source |
| 40: | suction module | 42: | vacuum suction line |
| 50: | optical sensor | 60: | auxiliary optical sensor |
| 70: | touch sensor | S: | suction space |

## Claims

1. A skin care treatment device, comprising:
a main body;
a suction header having, at an end portion protruding outward from the main body, a suction port configured to contact skin;
a light irradiation module configured to irradiate, toward a user's skin contacting the suction port, light generated when power is applied;
a suction module provided inside the main body and configured to apply, toward the suction port, negative pressure for raising a treatment site of the skin contacting the suction port when power is applied;
at least one optical sensor configured to sense and measure light introduced inward through an optical path formed at an end portion of the suction port contacting the skin; and
a control unit electrically connected to the light irradiation module and the suction module, respectively,
wherein the control unit is configured to determine a contact state between the suction port and the skin based on a measured value sensed by the optical sensor and selectively control an operation timing of the suction module.

2. The skin care treatment device of claim 1, wherein the optical sensor is a color sensor configured to sense a skin color of a skin region related to light introduced through the optical path and convert color information into an electrical signal.

3. The skin care treatment device of claim 1, wherein the optical sensor is an illuminance sensor configured to sense brightness of light introduced through the optical path and convert the brightness into an electrical signal according to an amount of the sensed light.

4. The skin care treatment device of claim 1, further comprising:
at least one auxiliary optical sensor provided on an inner wall of the suction port corresponding to a suction space formed inside the suction port,
wherein the auxiliary optical sensor is one of a color sensor configured to sense a skin color of a skin region related to light introduced into the suction space and convert color information into an electrical signal, and an illuminance sensor configured to sense brightness of light introduced into the suction space and convert the brightness into an electrical signal according to an amount of the sensed light.

5. The skin care treatment device of claim 2 or 4, wherein the control unit is configured to control an output intensity of the light irradiation module based on the skin color sensed and measured by the color sensor.

6. The skin care treatment device of claim 1, wherein the suction port comprises at least one touch sensor configured to generate a contact signal upon contact with the skin at an end portion of the suction port and transmit the generated contact signal to the control unit.
